# EUROPEAN PATENT APPLICATION

(11) **EP 0 980 911 A2**
(43) Date of publication of application: **23.02.2000**
(21) Application number: 98110192.6
(22) Date of filing: 04.06.1998
(51) Int. Cl.: C12N 15/55, C12N 9/16

(54) **Phosphodiesterase**

(71) Applicant: BAYER AG, 51368 Leverkusen (DE)
(72) Inventor: Wunder, Frank Dr., 42115 Wuppertal (DE)

(57) **Abstract**

The present invention relates to a new phosphodiesterase.

## Description

Phosphodiesterases are important regulators of the cellular response to different stimuli like hormones or neurotransmitters. During the last years it has become obvious that several cyclic nucleotide phosphodiesterases exist. These enzymes have been grouped into seven different families (PDE1-PDE7) according to their distinctive catalytic and regulatory properties.

PDE1 is a Ca²⁺/CaM-activated phosphodiesterase, which hydrolyzes preferentially cGMP but also cAMP. At least three different genes (PDE1A, PDE1B and PDE1C) coding for Ca²⁺/CaM-activated phosphodiesterases have now been cloned.

The cGMP-stimulated PDEs (PDE2) catalyze the degradation of cAMP and cGMP, the enzymatic activity is allosterically regulated by the binding of cGMP. So far only one gene (with at least three different splice variants) coding for PDE2 has been identified. A PDE2 cDNA was originally cloned from bovine adrenal gland, corresponding cDNAs from rat and human tissues have recently been reported.

PDE3 is known as the cGMP-inhibited PDE. It catalyzes the breakdown of both cAMP and cGMP, but with a higher Vₘₐₓ for cAMP. Its activity is inhibited by cGMP which presumably is due to a competition at the catalytic site.

The PDE4 isoenzymes are the cAMP-specific phosphodiesterases which preferentially hydrolyze cAMP. Up to now four different PDE4 genes have been cloned (PDE4A, PDE4B, PDE4C and PDE4D). By alternative splicing of the corresponding mRNAs a huge number of proteins with different N-termini are generated.

PDE5 is the cGMP-specific (or cGMP-binding) PDE, which preferentially hydrolyzes cGMP. So far only one gene has been identified, the corresponding cDNA was cloned from bovine lung.

Two relatively new classes are the PDE6 and PDE7 families, the cGMP-specific photoreceptor PDE (PDE6) and the cAMP-specific, rolipram-insensitive PDE (PDE7). Genes coding for PDE6 (PDE6A, PDE6B and PDE6C) have been cloned from retina, PDE7 was originally cloned from an glioblastoma cDNA library.

In order to identify new genes coding for phosphodiesterases a data base search using the regulatory and catalytic domains of the cGMP-stimulated and cGMP-specific phosphodiesterases was performed.

Beside known phosphodiesterase sequences, an *Expressed Sequence Tag* (EST RS7341) similar but not identical to the known cGMP-stimulated and the cGMP-specific phosphodiesterases could be identified. This EST was amplified by PCR (polymerase chain reaction), sequenced and used as a probe to screen different cDNA-libraries. From a rat brain library several overlapping cDNAs could be isolated. The complete coding region of the new phosphodiesterase gene was derived from two overlapping clones (rb20 and rb29). To verify the sequence, the cDNA construct was sequenced completely in both directions.

The nucleotides and deduced amino acid sequence are shown in Fig. 1. The complete sequence consists of 4076 nucleotides. A translation initiation codon at position 995 defines the beginning of an open reading frame of 2322 nucleotides. This would predict a protein of 774 amino acids with a molecular weight of 87759. Since the first putative initiation codon does not occur within a favorable consensus sequence for translation initiation, initiation at positions 1049, 1073, 1094 or 1175 might also be possible.

The derived protein sequence shows highest similarities to the cGMP-stimulated and to the cGMP-specific phosphodiesterases. Within the catalytic region an identity of 42% (over 262 aa) to PDE2 and an identity of 45% (over 272 aa) to PDE5 is found. Within the regulatory region an identity of 34% (272 aa) to PDE2 and of 46% (158 aa) to PDE5 is found. Within these regions PDE2 and PDE5 are 38% (catalytic) and 42% (regulatory) identical, respectively.

To characterize the expression pattern of the newly identified phosphodiesterase, Northern blots and *in situ*-hybridizations with rat and human tissues were performed. The mRNA was found to be mainly expressed in adult and embryonic (E19) central nervous system (CNS), in the adrenal gland and in testis. Within the CNS of adult rats the transcript is broadly expressed. However, highest expression could be found in corpus striatum, hypophysis, cerebellum and hippocampus.

### References:

Beavo, J.A. (1995) Cyclic nucleotide phosphodiesterases: Functional implications of multiple isoforms. Physiol. Rev. **75**, 725-748
Beavo, J.A., Conti, M. and Heaslip, R.J. (1994) Multiple cyclic nucleotide phosphodiesterases. Mol. Pharmacol. **46**, 399-405
Burns, F., Zhao, A.Z. and Beavo J.A. (1996) Cyclic nucleotide phosphodiesterases: Gene complexity, regulation by phosphorylation, and physiological implications. Adv. Pharmacol. **36**, 29-48
Rybalkin S.D. and Beavo, J.A. (1996) Multiplicity within cyclic nucleotide phosphodiesterases. Biochem. Soc. Trans. **24**, 1005-1009
Figure 1: Nucleotide and derived amino acid sequence of the newly identified phosphodiesterase from rat brain. Nucleotides are numbered at the end of each line. The upsteam in-frame stop codon and the five putative initiation codons are underlined. The translation termination signal at nucleotide 3317 is marked by an asterisk.

AAAAAGTGCCTGAGTCCGGGTATATTCTTCAGTCTTAGAGGAGCCTTGAGGCAATACCGA 60
CGCCCTTCCTCAATCATCGCCCATACCCAGAGTTGAAACCTAATTGCTGAGAGCCAGTGT 120
GCTCTGGGCTAGACTTGGTCTCATAGCGGAAACGGATGTCAGGACACACTGGGAACCAGG 180
GATGTGCTGATGGGAGGCACCGAAAGAAAAGGGAAACTCAGACCCATCTGAACTTGGCAG 240
CTTGCATAGGGGCTGTTTTTGGCTGGGCCTGCTGTACAGACACAGACTGGGGCGGGAGGG 300
CTGTAAGTTGGAGGCCAGCATAGGCTGTAAAGGAAAGCACGTATCAAACTTAATGTGGTG 360
CAGGTGTACTATACAGGCAGACAGACTTTGATGGCTGAAGATGATGCAGGACCCAAAATA 420
GAAATGCTTACAAAGATGTAGTTTATTTAGAGAAAGGATGCAACATTGAAACAACATGCC 480
AGGGATACATCATTGAAACTACAGGCCTGCCCTGCTGGATCTGGAGAGGTGGGCAGAGTT 540
GCCACAGCCGACAAGCCACTCTAGATCCTATGTCCTACACTGTGGATATTATACTGGCAT 600
TGTCCTATCTATAGCCAGCACCAGTGGCTGGTCCCTTGGGGTCTCACTGAGACTAGCTGA 660
TAGCCGTCACTATCACTTCAGCAATAAATGCTGGCCACAGGGGTGTGTGCTGCTGTGTGC 720
TGCTTGCACTAGCCTTTGTAGGAAGTGGCCAAGCCCTTGCATGACTGCTGGGACCTCTCC 780
TCAGAGCACATGCACTAAGGGGGAGGGGTTGGGGCGGGGTGGTGCAGGGCAGTGTCTGTG 840
TGGCCTTTCTCCTTCTGCCTCTTTGTAACCCATTAGAGTCCGTAGAGTTGACTGTGCTTG 900
TGTGTAACCAAGTATCTATTTTATAATCTTAATACAATGTACAAAGAGTTGCTTGTGGTA 960
TTTAGTCTTCCCAAATTTATTCTCTCGATGTTTTATGTTTAACCTTTCCAGTCTTGAAAA 1020
M F N L S S L E K

ATCAAGACCCTCAGACCTCCTAAGAAGTATGTCAAGAGCGAACGCATGTGCTATGTCTTG 1080
S R P S D L L R S M S R A N A C A M S W

GTGCCCATTCCAGATGCTAACTCTTTCTCTCCTTGCTTCTCTACCCCCAGATGAACCATC 1140
C P F Q M L T L S L L A S L P P D E P S

TCCTAAGGAAGTCAGCAGGTACCAGGACACGAACATGCAGGGAGTCGTGTACGAGCTGAA 1200
P K E V S R Y Q D T N M Q G V V Y E L N

CAGCTACATAGAGCAGCGCCTGGACACCGGCGGGGACAACCACCTGCTCCTGTACGAGCT 1260
S Y I E Q R L D T G G D N H L L L Y E L

AAGCAGTATCATCAGGATAGCCACAAAAGCCGACGGATTTGCACTGTACTTCCTTGGAGA 1320
S S I I R I A T K A D G F A L Y F L G E

GTGCAATAATAGTCTGTGTGTCTTCACACCACCCGGAATGAAGGAAGGTCAACCCCGTCT 1380
C N N S L C V F T P P G M K E G Q P R L

CATCCCCGCAGGGCCCATCACCCAGGGCACCACCATCTCTGCCTATGTGGCCAAGTCTAG 1440
I P A G P I T Q G T T I S A Y V A K S R

GAAGACCCTGCTGGTAGAGGACATCCTTGGGGATGAGCGATTTCCCAGAGGCACTGGTCT 1500
K T L L V E D I L G D E R F P R G T G L

GGAGTCAGGAACCCGAATCCAGTCTGTCCTTTGCTTGCCTATTGTCACTGCCATTGGAGA 1560
E S G T R I Q S V L C L P I V T A I G D

CTTGATTGGCATCCTTGAACTGTACAGGCACTGGGGCAAAGAGGCCTTCTGCCTCAGCCA 1620
L I G I L E L Y R H W G K E A F C L S H

TCAGGAGGTTGCAACCGCCAATCTCGCTTGGGCTTCCGTAGCAATACACCAGGTGCAGGT 1680
Q E V A T A N L A W A S V A I H Q V Q V

GTGCAGAGGTCTCGCCAAGCAGACCGAACTGAATGACTTCCTGCTCGATGTATCAAAGAC 1740
C R G L A K Q T E L N D F L L D V S K T

ATACTTTGATAACATAGTCGCCATAGACTCTCTACTTGAACACATCATGATATATGCAAA 1800
Y F D N I V A I D S L L E H I M I Y A K

AAATCTAGTGAACGCCGACCGCTGCGCGCTCTTCCAGGTGGACCACAAGAACAAGGAGCT 1860
N L V N A D R C A L F Q V D H K N K E L

GTACTCGGACCTGTTTGACATTGGGGAGGAGAAGGAGGGGAAGCCCGTCTTCAAGAAGAC 1920
Y S D L F D I G E E K E G K P V F K K T

CAAGGAGATCAGATTTTCCATTGAGAAAGGGATTGCTGGTCAAGTGGCAAGAACGGGAGA 1980
K E I R F S I E K G I A G Q V A R T G E

AGTCCTGAACATTCCTGATGCCTACGCAGACCCGCGCTTTAACAGGGAGGTGGACCTGTA 2040
V L N I P D A Y A D P R F N R E V D L Y

CACAGGCTATACCACGCGGAACATTCTGTGTATGCCCATAGTGAGCCGCGGCAGCGTGAT 2100
T G Y T T R N I L C M P I V S R G S V I

CGGTGTGGTGCAAATGGTTAACAAGATCAGCGGCAGCGCCTTCTCCAAGACGGATGAGAA 2160
G V V Q M V N K I S G S A F S K T D E N

CAACTTCAAGATGTTTGCTGTCTTCTGCGCTCTGGCCCTGCACTGCGCTAACATGTACCA 2220
N F K M F A V F C A L A L H C A N M Y H

CAGGATCCGCCACTCAGAGTGCATCTACAGGGTTACCATGGAGAAGCTGTCTTACCACAG 2280
R I R H S E C I Y R V T M E K L S Y H S

CATCTGCACCTCTGAGGAATGGCAAGGCCTCATGCACTTCAACTTGCCAGCACGCATCTG 2340
I C T S E E W Q G L M H F N L P A R I C

CCGGGACATCGAGCTATTCCACTTTGACATTGGTCCTTTCGAGAACATGTGGCCTGGGAT 2400
R D I E L F H F D I G P F E N M W P G I

CTTTGTCTACATGATCCATCGGTCTTGTGGGACATCCTGTTTTGAACTTGAAAAATTGTG 2460
F V Y M I H R S C G T S C F E L E K L C

CCGTTTTATCATGTCTGTGAAGAAGAACTATAGGCGGGTTCCTTACCACAACTGGAAGCA 2520
R F I M S V K K N Y R R V P Y H N W K H

TGCAGTCACGGTGGCGCACTGCATGTACGCCATACTTCAAAACAACAATGGCCTCTTCAC 2580
A V T V A H C M Y A I L Q N N N G L F T

AGACCTTGAGCGCAAAGGCCTGCTAATTGCCTGTCTGTGCCATGACCTGGACCACAGGGG 2640
D L E R K G L L I A C L C H D L D H R G

CTTCAGTAACAGCTACCTGCAGAAATTCGACCACCCCCTGGCTGCGTTGTACTCCACCTC 2700
F S N S Y L Q K F D H P L A A L Y S T S

CACCATGGAGCAACACCACTTCTCCCAGACGGTGTCCATCCTCCAGCTGGAAGGACACAA 2760
T M E Q H H F S Q T V S I L Q L E G H N

CATCTTCTCCACCCTGAGCTCCAGCGAGTACGAGCAGGTGCTGGAGATCATCCGCAAAGC 2820
I F S T L S S S E Y E Q V L E I I R K A

CATCATCGCCACTGACCTCGCACTGTACTTTGGGAACAGGAAGCAGTTGGAGGAGATGTA 2880

I I A T D L A L Y F G N R K Q L E E M Y

CCAGACAGGGTCGCTGAACCTCCACAACCAGTCCCATCGAGACCGCGTCATCGGCTTGAT 2940
Q T G S L N L H N Q S H R D R V I G L M

GATGACTGCCTGCGATCTTTGCTCTGTGACGAAACTATGGCCAGTTACAAAATTGACAGC 3000
M T A C D L C S V T K L W P V T K L T A

AAATGATATATATGCAGAGTTCTGGGCTGAGGGGGATGAGATGAAGAAGTTGGGGATACA 3060
N D I Y A E F W A E G D E M K K L G I Q

GCCCATCCCTATGATGGACAGAGACAAGCGAGATGAAGTCCCTCAAGGACAGCTTGGATT 3120
P I P M M D R D K R D E V P Q G Q L G F

CTACAATGCTGTGGCCATCCCCTGCTATACCACCCTGACGCAGATCCTCCCACCCACAGA 3180
Y N A V A I P C Y T T L T Q I L P P T E

GCCTCTGCTGAAGGCCTGCAGGGATAACCTCAATCAGTGGGAGAAGGTAATTCGAGGGGA 3240
P L L K A C R D N L N Q W E K V I R G E

AGAGACAGCAATGTGGATTTCAGGCCCAGCAACTAGCAAAAGCACATCTGAGAAGCCGAC 3300
E T A M W I S G P A T S K S T S E K P T

CAGGAAGGTCGATGACTGATCCTGAGGTGATGTCTGCCTAGCAACTGACTCAACCTGCTT 3360
R K V D D *

CTGTGACTTCGTTCTTTTTATTTTTATTTTTTTAACGGGGTGAAAACCTCTCTCAGAAGG 3420
TACCGTCGCATATCCATGTGAAGCAGATGACTCCCTGCGCACACCTCGGACCGTGAGCAA 3480
CCCGGGCTCCACCGTGTTCAGACGTCGGCTATTCCATGGCTCCGCCTGACCCCCGAATGC 3540
CATTTGCTACCAGGCCAGAACTGCGCTGGCTGGAGGGGGCAGAGACGACAGGAGGGGTTC 3600
TTACCTGCATCCTTCCATGAGGGTGTGGTTCTGTGTTTCATCTCTAACAGAGATGCTACT 3660
GCTTGGTGGCGTTTGTTAGAAATGGGACACATGCCCCTGTCGTGAAGTTTACATGTGACC 3720
TTCTTGTAGGTAACTTGAGTTCGTAGCCTGGGACCCCTGTAATGAAGGTTACAGTCCACA 3780
GGTGATAGAGAAATTCAAGCTGTAAGTTACAGGTGCACTACAAGTGTGTTCATTCAGTTT 3840
ACCTGGGGGCATGGAGGTGAGTCAGCTCCACGAGGAAGGAAGCATACCTCTGCCCTCATC 3900
AAGGGGACACAGGGTACATCCCAGGCATCAGAGAACTGCAGCTCACCTCAAACCATGTCA 3960
AAGAATTAAAACACACCCCCATCCCCTCACTGTAGCCTTTGGCAACTTCGCCAAACCCTT 4020
CACACAAAGAAAATAAAAGTAAGGCGTATAAATTTCCTCCAGCAAGCAAATCTTGT 4076

Figure 2: Derived protein sequence of the new phosphodiesterase.

MFNLSSLEKS RPSDLLRSMS RANACAMSWC PFQMLTLSLL ASLPPDEPSP 50
KEVSRYQDTN MQGVVYELNS YIEQRLDTGG DNHLLLYELS SIIRIATKAD 100
GFALYFLGEC NNSLCVFTPP GMKEGQPRLI PAGPITQGTT ISAYVAKSRK 150
TLLVEDILGD ERFPRGTGLE SGTRIQSVLC LPIVTAIGDL IGILELYRHW 200
GKEAFCLSHQ EVATANLAWA SVAIHQVQVC RGLAKQTELN DFLLDVSKTY 250
FDNIVAIDSL LEHIMIYAKN LVNADRCALF QVDHKNKELY SDLFDIGEEK 300
EGKPVFKKTK EIRFSIEKGI AGQVARTGEV LNIPDAYADP RFNREVDLYT 350
GYTTRNILCM PIVSRGSVIG VVQMVNKISG SAFSKTDENN FKMFAVFCAL 400
ALHCANMYHR IRHSECIYRV TMEKLSYHSI CTSEEWQGLM HFNLPARICR 450
DIELFHFDIG PFENMWPGIF VYMIHRSCGT SCFELEKLCR FIMSVKKNYR 500
RVPYHNWKHA VTVAHCMYAI LQNNNGLFTD LERKGLLIAC LCHDLDHRGF 550
SNSYLQKFDH PLAALYSTST MEQHHFSQTV SILQLEGHNI FSTLSSSEYE 600
QVLEIIRKAI IATDLALYFG NRKQLEEMYQ TGSLNLHNQS HRDRVIGLMM 650
TACDLCSVTK LWPVTKLTAN DIYAEFWAEG DEMKKLGIQP IPMMDRDKRD 700
EVPQGQLGFY NAVAIPCYTT LTQILPPTEP LLKACRDNLN QWEKVIRGEE 750
TAMWISGPAT SKSTSEKPTR KVDD 774

## Claims

1. A DNA having the sequence of the DNA identified as Seq IDNo1.

2. A protein having the sequence of the protein identified as Seq IDNo2.


SEQUENCE LISTING

(1) GENERAL INFORMATION:

(i) APPLICANT:
(A) NAME: Bayer AG
(B) STREET: Bayerwerk
(C) CITY: Leverkusen
(E) COUNTRY: Germany
(F) POSTAL CODE (ZIP): 51368
(G) TELEPHONE: 0214-3061455
(H) TELEFAX: 0214-303482

(ii) TITLE OF INVENTION: Phosphodiesterase

(iii) NUMBER OF SEQUENCES: 2

(iv) COMPUTER READABLE FORM:
(A) MEDIUM TYPE: Floppy disk
(B) COMPUTER: IBM PC compatible
(C) OPERATING SYSTEM: PC-DOS/MS-DOS
(D) SOFTWARE: PatentIn Release #1.0, Version #1.30B (EPO)

(2) INFORMATION FOR SEQ ID NO: 1:

(i) SEQUENCE CHARACTERISTICS:
(A) LENGTH: 4076 base pairs
(B) TYPE: nucleic acid
(C) STRANDEDNESS: single
(D) TOPOLOGY: linear

(ii) MOLECULE TYPE: DNA (genomic)

(iii) HYPOTHETICAL: NO

(iv) ANTI-SENSE: NO

(vi) ORIGINAL SOURCE:
(A) ORGANISM: Phosphodiesterase
(C) INDIVIDUAL ISOLATE: rat

(xi) SEQUENCE DESCRIPTION: SEQ ID NO: 1:

AAAAAGTGCC TGAGTCCGGG TATATTCTTC AGTCTTAGAG GAGCCTTGAG GCAATACCGA 60

CGCCCTTCCT CAATCATCGC CCATACCCAG AGTTGAAACC TAATTGCTGA GAGCCAGTGT 120

GCTCTGGGCT AGACTTGGTC TCATAGCGGA AACGGATGTC AGGACACACT GGGAACCAGG 180

GATGTGCTGA TGGGAGGCAC CGAAAGAAAA GGGAAACTCA GACCCATCTG AACTTGGCAG 240

CTTGCATAGG GGCTGTTTTT GGCTGGGCCT GCTGTACAGA CACAGACTGG GGCGGGAGGG 300
CTGTAAGTTG GAGGCCAGCA TAGGCTGTAA AGGAAAGCAC GTATCAAACT TAATGTGGTG 360
CAGGTGTACT ATACAGGCAG ACAGACTTTG ATGGCTGAAG ATGATGCAGG ACCCAAAATA 420
GAAATGCTTA CAAAGATGTA GTTTATTTAG AGAAAGGATG CAACATTGAA ACAACATGCC 480
AGGGATACAT CATTGAAACT ACAGGCCTGC CCTGCTGGAT CTGGAGAGGT GGGCAGAGTT 540
GCCACAGCCG ACAAGCCACT CTAGATCCTA TGTCCTACAC TGTGGATATT ATACTGGCAT 600
TGTCCTATCT ATAGCCAGCA CCAGTGGCTG GTCCCTTGGG GTCTCACTGA GACTAGCTGA 660
TAGCCGTCAC TATCACTTCA GCAATAAATG CTGGCCACAG GGGTGTGTGC TGCTGTGTGC 720
TGCTTGCACT AGCCTTTGTA GGAAGTGGCC AAGCCCTTGC ATGACTGCTG GGACCTCTCC 780
TCAGAGCACA TGCACTAAGG GGGAGGGGTT GGGGCGGGGT GGTGCAGGGC AGTGTCTGTG 840
TGGCCTTTCT CCTTCTGCCT CTTTGTAACC CATTAGAGTC CGTAGAGTTG ACTGTGCTTG 900
TGTGTAACCA AGTATCTATT TTATAATCTT AATACAATGT ACAAAGAGTT GCTTGTGGTA 960
TTTAGTCTTC CCAAATTTAT TCTCTCGATG TTTTATGTTT AACCTTTCCA GTCTTGAAAA 1020
ATCAAGACCC TCAGACCTCC TAAGAAGTAT GTCAAGAGCG AACGCATGTG CTATGTCTTG 1080
GTGCCCATTC CAGATGCTAA CTCTTTCTCT CCTTGCTTCT CTACCCCCAG ATGAACCATC 1140
TCCTAAGGAA GTCAGCAGGT ACCAGGACAC GAACATGCAG GGAGTCGTGT ACGAGCTGAA 1200
CAGCTACATA GAGCAGCGCC TGGACACCGG CGGGGACAAC CACCTGCTCC TGTACGAGCT 1260
AAGCAGTATC ATCAGGATAG CCACAAAAGC CGACGGATTT GCACTGTACT TCCTTGGAGA 1320
GTGCAATAAT AGTCTGTGTG TCTTCACACC ACCCGGAATG AAGGAAGGTC AACCCCGTCT 1380
CATCCCCGCA GGGCCCATCA CCCAGGGCAC CACCATCTCT GCCTATGTGG CCAAGTCTAG 1440
GAAGACCCTG CTGGTAGAGG ACATCCTTGG GGATGAGCGA TTTCCCAGAG GCACTGGTCT 1500
GGAGTCAGGA ACCCGAATCC AGTCTGTCCT TTGCTTGCCT ATTGTCACTG CCATTGGAGA 1560
CTTGATTGGC ATCCTTGAAC TGTACAGGCA CTGGGGCAAA GAGGCCTTCT GCCTCAGCCA 1620
TCAGGAGGTT GCAACCGCCA ATCTCGCTTG GGCTTCCGTA GCAATACACC AGGTGCAGGT 1680
GTGCAGAGGT CTCGCCAAGC AGACCGAACT GAATGACTTC CTGCTCGATG TATCAAAGAC 1740
ATACTTTGAT AACATAGTCG CCATAGACTC TCTACTTGAA CACATCATGA TATATGCAAA 1800
AAATCTAGTG AACGCCGACC GCTGCGCGCT CTTCCAGGTG GACCACAAGA ACAAGGAGCT 1860
GTACTCGGAC CTGTTTGACA TTGGGGAGGA GAAGGAGGGG AAGCCCGTCT TCAAGAAGAC 1920

CAAGGAGATC AGATTTTCCA TTGAGAAAGG GATTGCTGGT CAAGTGGCAA GAACGGGAGA 1980

AGTCCTGAAC ATTCCTGATG CCTACGCAGA CCCGCGCTTT AACAGGGAGG TGGACCTGTA 2040

CACAGGCTAT ACCACGCGGA ACATTCTGTG TATGCCCATA GTGAGCCGCG GCAGCGTGAT 2100

CGGTGTGGTG CAAATGGTTA ACAAGATCAG CGGCAGCGCC TTCTCCAAGA CGGATGAGAA 2160

CAACTTCAAG ATGTTTGCTG TCTTCTGCGC TCTGGCCCTG CACTGCGCTA ACATGTACCA 2220

CAGGATCCGC CACTCAGAGT GCATCTACAG GGTTACCATG GAGAAGCTGT CTTACCACAG 2280

CATCTGCACC TCTGAGGAAT GGCAAGGCCT CATGCACTTC AACTTGCCAG CACGCATCTG 2340

CCGGGACATC GAGCTATTCC ACTTTGACAT TGGTCCTTTC GAGAACATGT GGCCTGGGAT 2400

CTTTGTCTAC ATGATCCATC GGTCTTGTGG GACATCCTGT TTTGAACTTG AAAAATTGTG 2460

CCGTTTTATC ATGTCTGTGA AGAAGAACTA TAGGCGGGTT CCTTACCACA ACTGGAAGCA 2520

TGCAGTCACG GTGGCGCACT GCATGTACGC CATACTTCAA AACAACAATG GCCTCTTCAC 2580

AGACCTTGAG CGCAAAGGCC TGCTAATTGC CTGTCTGTGC CATGACCTGG ACCACAGGGG 2640

CTTCAGTAAC AGCTACCTGC AGAAATTCGA CCACCCCCTG GCTGCGTTGT ACTCCACCTC 2700

CACCATGGAG CAACACCACT TCTCCCAGAC GGTGTCCATC CTCCAGCTGG AAGGACACAA 2760

CATCTTCTCC ACCCTGAGCT CCAGCGAGTA CGAGCAGGTG CTGGAGATCA TCCGCAAAGC 2820

CATCATCGCC ACTGACCTCG CACTGTACTT TGGGAACAGG AAGCAGTTGG AGGAGATGTA 2880

CCAGACAGGG TCGCTGAACC TCCACAACCA GTCCCATCGA GACCGCGTCA TCGGCTTGAT 2940

GATGACTGCC TGCGATCTTT GCTCTGTGAC GAAACTATGG CCAGTTACAA AATTGACAGC 3000

AAATGATATA TATGCAGAGT TCTGGGCTGA GGGGGATGAG ATGAAGAAGT TGGGGATACA 3060

GCCCATCCCT ATGATGGACA GAGACAAGCG AGATGAAGTC CCTCAAGGAC AGCTTGGATT 3120

CTACAATGCT GTGGCCATCC CCTGCTATAC CACCCTGACG CAGATCCTCC CACCCACAGA 3180

GCCTCTGCTG AAGGCCTGCA GGGATAACCT CAATCAGTGG GAGAAGGTAA TTCGAGGGGA 3240

AGAGACAGCA ATGTGGATTT CAGGCCCAGC AACTAGCAAA AGCACATCTG AGAAGCCGAC 3300

CAGGAAGGTC GATGACTGAT CCTGAGGTGA TGTCTGCCTA GCAACTGACT CAACCTGCTT 3360

CTGTGACTTC GTTCTTTTTA TTTTTATTTT TTTAACGGGG TGAAAACCTC TCTCAGAAGG 3420

TACCGTCGCA TATCCATGTG AAGCAGATGA CTCCCTGCGC ACACCTCGGA CCGTGAGCAA 3480

CCCGGGCTCC ACCGTGTTCA GACGTCGGCT ATTCCATGGC TCCGCCTGAC CCCCGAATGC 3540

CATTTGCTAC CAGGCCAGAA CTGCGCTGGC TGGAGGGGGC AGAGACGACA GGAGGGGTTC 3600

TTACCTGCAT CCTTCCATGA GGGTGTGGTT CTGTGTTTCA TCTCTAACAG AGATGCTACT 3660

GCTTGGTGGC GTTTGTTAGA AATGGGACAC ATGCCCCTGT CGTGAAGTTT ACATGTGACC 3720

TTCTTGTAGG TAACTTGAGT TCGTAGCCTG GGACCCCTGT AATGAAGGTT ACAGTCCACA 3780

GGTGATAGAG AAATTCAAGC TGTAAGTTAC AGGTGCACTA CAAGTGTGTT CATTCAGTTT 3840

ACCTGGGGGC ATGGAGGTGA GTCAGCTCCA CGAGGAAGGA AGCATACCTC TGCCCTCATC 3900

AAGGGGACAC AGGGTACATC CCAGGCATCA GAGAACTGCA GCTCACCTCA AACCATGTCA 3960

AAGAATTAAA ACACACCCCC ATCCCCTCAC TGTAGCCTTT GGCAACTTCG CCAAACCCTT 4020

CACACAAAGA AAATAAAAGT AAGGCGTATA AATTTCCTCC AGCAAGCAAA TCTTGT 4076

(2) INFORMATION FOR SEQ ID NO: 2:

(i) SEQUENCE CHARACTERISTICS:
(A) LENGTH: 774 amino acids
(B) TYPE: amino acid
(C) STRANDEDNESS: single
(D) TOPOLOGY: linear

(ii) MOLECULE TYPE: protein

(iii) HYPOTHETICAL: NO

(iv) ANTI-SENSE: NO

(vi) ORIGINAL SOURCE:
(A) ORGANISM: Phosphodiesterase
(C) INDIVIDUAL ISOLATE: rat, rat brain

(xi) SEQUENCE DESCRIPTION: SEQ ID NO: 2:

Met Phe Asn Leu Ser Ser Leu Glu Lys Ser Arg Pro Ser Asp Leu Leu
1               5                   10                  15

Arg Ser Met Ser Arg Ala Asn Ala Cys Ala Met Ser Trp Cys Pro Phe
            20                  25                  30

Gln Met Leu Thr Leu Ser Leu Leu Ala Ser Leu Pro Pro Asp Glu Pro
        35                  40                  45

Ser Pro Lys Glu Val Ser Arg Tyr Gln Asp Thr Asn Met Gln Gly Val
    50                  55                  60

Val Tyr Glu Leu Asn Ser Tyr Ile Glu Gln Arg Leu Asp Thr Gly Gly
65                  70                  75                  80

Asp Asn His Leu Leu Leu Tyr Glu Leu Ser Ser Ile Ile Arg Ile Ala
                85                  90                  95

Thr Lys Ala Asp Gly Phe Ala Leu Tyr Phe Leu Gly Glu Cys Asn Asn
            100                 105                 110

Ser Leu Cys Val Phe Thr Pro Pro Gly Met Lys Glu Gly Gln Pro Arg
        115                 120                 125

Leu Ile Pro Ala Gly Pro Ile Thr Gln Gly Thr Thr Ile Ser Ala Tyr
    130                 135                 140

Val Ala Lys Ser Arg Lys Thr Leu Leu Val Glu Asp Ile Leu Gly Asp
145                 150                 155                 160

Glu Arg Phe Pro Arg Gly Thr Gly Leu Glu Ser Gly Thr Arg Ile Gln
                165                 170                 175

Ser Val Leu Cys Leu Pro Ile Val Thr Ala Ile Gly Asp Leu Ile Gly
            180                 185                 190

Ile Leu Glu Leu Tyr Arg His Trp Gly Lys Glu Ala Phe Cys Leu Ser
        195                 200                 205

His Gln Glu Val Ala Thr Ala Asn Leu Ala Trp Ala Ser Val Ala Ile
    210                 215                 220

His Gln Val Gln Val Cys Arg Gly Leu Ala Lys Gln Thr Glu Leu Asn
225                 230                 235                 240

Asp Phe Leu Leu Asp Val Ser Lys Thr Tyr Phe Asp Asn Ile Val Ala
                245                 250                 255

Ile Asp Ser Leu Leu Glu His Ile Met Ile Tyr Ala Lys Asn Leu Val
            260                 265                 270

Asn Ala Asp Arg Cys Ala Leu Phe Gln Val Asp His Lys Asn Lys Glu
        275                 280                 285

Leu Tyr Ser Asp Leu Phe Asp Ile Gly Glu Glu Lys Glu Gly Lys Pro
    290                 295                 300

Val Phe Lys Lys Thr Lys Glu Ile Arg Phe Ser Ile Glu Lys Gly Ile
305                 310                 315                 320

Ala Gly Gln Val Ala Arg Thr Gly Glu Val Leu Asn Ile Pro Asp Ala
                325                 330                 335

Tyr Ala Asp Pro Arg Phe Asn Arg Glu Val Asp Leu Tyr Thr Gly Tyr
            340                 345                 350

Thr Thr Arg Asn Ile Leu Cys Met Pro Ile Val Ser Arg Gly Ser Val
        355                 360                 365

Ile Gly Val Val Gln Met Val Asn Lys Ile Ser Gly Ser Ala Phe Ser

370                 375                 380

Lys Thr Asp Glu Asn Asn Phe Lys Met Phe Ala Val Phe Cys Ala Leu
385                 390                 395                 400

Ala Leu His Cys Ala Asn Met Tyr His Arg Ile Arg His Ser Glu Cys
                405                 410                 415

Ile Tyr Arg Val Thr Met Glu Lys Leu Ser Tyr His Ser Ile Cys Thr
            420                 425                 430

Ser Glu Glu Trp Gln Gly Leu Met His Phe Asn Leu Pro Ala Arg Ile
        435                 440                 445

Cys Arg Asp Ile Glu Leu Phe His Phe Asp Ile Gly Pro Phe Glu Asn
    450                 455                 460

Met Trp Pro Gly Ile Phe Val Tyr Met Ile His Arg Ser Cys Gly Thr
465                 470                 475                 480

Ser Cys Phe Glu Leu Glu Lys Leu Cys Arg Phe Ile Met Ser Val Lys
                485                 490                 495

Lys Asn Tyr Arg Arg Val Pro Tyr His Asn Trp Lys His Ala Val Thr
            500                 505                 510

Val Ala His Cys Met Tyr Ala Ile Leu Gln Asn Asn Asn Gly Leu Phe
        515                 520                 525

Thr Asp Leu Glu Arg Lys Gly Leu Leu Ile Ala Cys Leu Cys His Asp
    530                 535                 540

Leu Asp His Arg Gly Phe Ser Asn Ser Tyr Leu Gln Lys Phe Asp His
545                 550                 555                 560

Pro Leu Ala Ala Leu Tyr Ser Thr Ser Thr Met Glu Gln His His Phe
                565                 570                 575

Ser Gln Thr Val Ser Ile Leu Gln Leu Glu Gly His Asn Ile Phe Ser
            580                 585                 590

Thr Leu Ser Ser Ser Glu Tyr Glu Gln Val Leu Glu Ile Ile Arg Lys
        595                 600                 605

Ala Ile Ile Ala Thr Asp Leu Ala Leu Tyr Phe Gly Asn Arg Lys Gln
    610                 615                 620

Leu Glu Glu Met Tyr Gln Thr Gly Ser Leu Asn Leu His Asn Gln Ser
625                 630                 635                 640

His Arg Asp Arg Val Ile Gly Leu Met Met Thr Ala Cys Asp Leu Cys
                645                 650                 655

Ser Val Thr Lys Leu Trp Pro Val Thr Lys Leu Thr Ala Asn Asp Ile
            660                 665                 670

Tyr Ala Glu Phe Trp Ala Glu Gly Asp Glu Met Lys Lys Leu Gly Ile
        675                 680                 685

Gln Pro Ile Pro Met Met Asp Arg Asp Lys Arg Asp Glu Val Pro Gln
    690                 695                 700

Gly Gln Leu Gly Phe Tyr Asn Ala Val Ala Ile Pro Cys Tyr Thr Thr
705                 710                 715                 720

Leu Thr Gln Ile Leu Pro Pro Thr Glu Pro Leu Leu Lys Ala Cys Arg
                725                 730                 735

Asp Asn Leu Asn Gln Trp Glu Lys Val Ile Arg Gly Glu Glu Thr Ala
            740                 745                 750

Met Trp Ile Ser Gly Pro Ala Thr Ser Lys Ser Thr Ser Glu Lys Pro
        755                 760                 765

Thr Arg Lys Val Asp Asp
    770